(19) Europäisches Patentamt
European Patent Office
Office européen des brevets

(11) **EP 3 308 703 B1**

(12) **EUROPEAN PATENT SPECIFICATION**

(45) Date of publication and mention of the grant of the patent:
**02.10.2019 Bulletin 2019/40**

(51) Int Cl.:
***A61B 5/04*** *(2006.01)*

(21) Application number: **16193225.6**

(22) Date of filing: **11.10.2016**

(54) **MAGNETOCARDIOGRAPHIC METHOD AND MAGNETOCARDIOGRAPHIC SYSTEM**

MAGNETKARDIOGRAPHIEVERFAHREN UND MAGNETKARDIOGRAPHIESYSTEM

MÉTHODE MAGNÉTOCARDIOGRAPHIE ET SYSTÈME MAGNÉTOCARDIOGRAPHIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**18.04.2018 Bulletin 2018/16**

(73) Proprietor: **Biomagnetik Park GmbH**
**21149 Hamburg (DE)**

(72) Inventors:
• **EHRLEN, Malte**
**20257 Hamburg (DE)**
• **PARK, Jai-Wun**
**21149 Hamburg (DE)**
• **LAM, Yat Yin**
**Hong Kong SAR (HK)**

(74) Representative: **Stüven, Ralf et al**
**Pohl & Partner**
**Patentanwälte**
**Kirchenhang 32 b**
**21073 Hamburg (DE)**

(56) References cited:
**DE-A1- 10 042 138**

• **HANNINEN H ET AL: "Detection of exercise induced myocardial ischemia by multichannel magnetocardiography in single vessel coronary artery disease", ANNALS OF NONINVASIVE ELECTROCARDIOLOGY, FUTURA PUB., ARMONK, US, vol. 5, no. 2, 1 April 2000 (2000-04-01), pages 147-157, XP002498639, ISSN: 1082-720X, DOI: 10.1111/J.1542-474X.2000.TB00380.X**
• **KONRAD BROCKMEIER ET AL: "Magnetocardiography and 32-Lead Potential Mapping.", JOURNAL OF CARDIOVASCULAR ELECTROPHYSIOLOGY., vol. 8, no. 6, 1 June 1997 (1997-06-01), pages 615-626, XP055358261, US ISSN: 1045-3873, DOI: 10.1111/j.1540-8167.1997.tb01824.x**
• **EBMEYER ET AL: "Predictive value of the magnetocardiogram for location of regional ischemia or infarction as detected by quantitative analysis of the coronary arteriogram", INTERNATIONAL CONGRESS SERIES, EXCERPTA MEDICA, AMSTERDAM, NL, vol. 1300, 1 June 2007 (2007-06-01), pages 463-467, XP022112931, ISSN: 0531-5131, DOI: 10.1016/J.ICS.2007.02.008**

EP 3 308 703 B1

## Description

The invention relates to a magnetocardiographic method and a magnetocardiographic system being arranged for carrying out the magnetocardiographic method. The invention further relates to a computer implemented method for identifying and quantifying myocardial disorder, and a data carrier comprising a computer program for carrying out the method.

Magnetocardiography (MCG) is a non-invasive method of recording magnetic fields generated by the electric activity of the heart, and is, i.a., used for clinical diagnosis of heart diseases, for example ischemic heart disease (IHD; see e.g. [1]-[8]). In WO 02/017769 A2, for example, a magnetic dipole model is used to localize ischemic cardiac tissue. The direction and extent of a displacement of the dipole during the ST segment of the sinus rhythm, superimposed on the hearts general outline, indicates the localization and extent of ischemia.

Brockmeier et al. [9] describe substantial changes in magnetocardiograms during the repolarization period of healthy subjects under stress conditions. Induced stress lead to changes in the field from dipolar to monopolar shape. These changes, however, were considered of nonpathologic origin.

Ebmeyer et al. [10] deal with the predictive value of magnetocardiograms for the location of regional ischemia or infarction. They generated current density vector (CVD) maps within the ST-T interval, and classified the CVD maps based on the dipolar or non-dipolar structure of the map.

DE 100 42 138 A1 relates to methods for automatic processing of biomagnetic field data, in particula macnetocardiographic data. The methods include, i.a., an estimation of the reciprocal arrangement of the maximum positive and negative extrema in each magnetic field distribution map, an estimation of the presence of additional extrema in each magnetic field distribution map and the duration of their existence during the repolarization process, and a calculation of the ratio of the maximum negative to the maximum positive extrema in each map.

However, prior art magnetocardiographic methods, especially in view of their application for diagnosing myocardial disorder, e.g, IHD, are still unsatisfactory.

It is therefore an object of the invention to provide an improved magnetocardiographic method, in particular in view of diagnosing ischemic heart disease.

In a first aspect the invention provides a magnetocardiographic method, the method comprising the following steps:

a) Measuring a component of the heart magnetic field at a plurality of positions above the heart of a subject using a plurality of magnetic field sensors during at least part of the cardiac cycle comprising the ST-T segment of the sinus rhythm of the heart, and

b) Determining, using the magnetic field data measured by the plurality of magnetic field sensors, the monopolarity of the magnetic field at at least one given point in time or over at least one given time period during the ST-T segment of the sinus rhythm of the heart, the monopolarity of the magnetic field representing a measure for the deviation of the magnetic field from a dipolar shape, wherein the determination of the monopolarity of the magnetic field is computer implemented, and wherein the monopolarity of the magnetic field is calculated by the following formula (1)

$$M = \frac{\left| \sum b_i \right|}{\sum \left| b_i \right|} \qquad (1),$$

wherein M is the monopolarity, $i$ the sensor index and $b_i$ the magnetic field gradient measured by the $i$-th magnetic field sensor.

The method according to the first aspect of the invention provides a diagnostic parameter for aiding in diagnosing a heart muscle tissue disorder, in particular for diagnosing the presence or absence of ischemic heart disease. It has been found that the monopolarity of the magnetic field, i.e. the degree of deviation of the magnetic field from a dipolar shape, in particular during the ST-T segment of the sinus rhythm of the heart provides a measure for indicating the presence and the severity of cardiac muscle tissue disorder. For this purpose, the monopolarity of the magnetic field of a subject's heart during the ST-T segment can be compared with the average monopolarity of persons with healthy heart muscle tissue.

The magnetic field generated by the current flow in heart muscle tissue, in particular during the ST-T segment, usually has essentially the shape of a dipole. The dipolar shape can, e.g., be visualized by magnetic isofield contour (isocontour) mapping, for example, using the z-component of the magnetic field. The magnetic field thus exhibits a minus peak, where the data measured by magnetic field sensors have their maximal negative value, and a positive peak, where data measured by magnetic field sensors at the same time reach their maximal positive value. Negative values correspond to magnetic field lines entering the body, positive values correspond to magnetic field lines leaving the body.

The monopolarity of the measured magnetic field can be determined at any time or over any time period during the whole cardiac cycle. It is, however, particular preferred to determine the monopolarity during at least part of the ST-T segment. i.e. at any one or several points in time during the ST-T segment and/or over any one or more intervals during the ST-T segment. In case of a plurality of intervals the intervals may be of equal or different length (duration), directly consecutive or not, or overlapping.

The term "monopolarity" in relation to a magnetic field generated by the heart relates to the degree to which a magnetic field deviates from an essentially dipolar shape, i.e. the degree of a shift from an essentially dipolar shape to a more monopolar shape. In particular, the monopolarity represents a measure for a distribution of positive and negative magnetic field sensor signals deviating from a dipolar pattern. A perfect dipolar shape of a magnetic field would result in an axially symmetric isocontour map plotted from measured data of a plurality of sensors, e.g. axial gradiometers, positioned directly above the magnetic field source and oriented in the z-axis using standard heart coordinates. A shift to a more monopolar shape, i.e. a magnetic field having a higher monopolarity, would result in an axially asymmetric isocontour map, where, for example, the majority of the sensors of a plurality of magnetic field sensors positioned directly above the magnetic field source would measure negative or positive values, and/or where the sum of (absolute) negative values is greater than the sum of the positive values, or vice versa. An example of a magnetic field having an essentially "monopolar" shape would, for example, be a magnetic field where all of a plurality of magnetic field sensors positioned directly above the magnetic field source measure only positive or only negative values. It is to be noted here that the term "essentially monopolar magnetic field" or "monopolar magnetic field" does not denote a magnetic monopole, but a magnetic field where one pole (negative or positive) is distributed over a larger area of heart tissue, such that no distinct peak (e.g. minus or plus peak) can be determined. The monopolarity can, for example, easily be determined by taking magnetic field data measured by magnetic field sensors at a specific point in time or over a specific time interval, and comparing, for example, the number of sensors with negative and positive data and/or comparing the magnitudes of the negative or positive data. An inherent offset of the sensors is, of course, taken into account. A perfectly dipolar magnetic field has no (or e.g. zero) monopolarity, whereas a magnetic field for which, for example, a larger number of sensors out of a plurality of sensors measuring the magnetic field measures a positive instead of a negative value, or vice versa, has a specific monopolarity (e.g. greater than zero). In particular, the terms "magnetic field having an essentially monopolar shape" or "essentially monopolar magnetic field" relates to a magnetic field where all of a plurality of magnetic field sensors measure either positive or negative values at a given point in time or over a given time interval. The preferably automatic quantification of the monopolarity of a magnetic field may be implemented in different ways.

The term "ST-T segment of the sinus rhythm of the heart" relates to a time interval of the cardiac cycle (heart beat, sinus rhythm), starting after the end of the S-wave and lasting until the end of the T-wave. The ST-T segment is also called the "ST interval". The ST segment represents the interval between the end of ventricular depolarization and the beginning of repolarization, the T-wave represents the repolarization of the ventricles. Although originally created for electrocardiography the terms for describing specific sections of an electrocardiogram (e.g. P wave, QRS complex, ST segment and T-wave) have been adopted for describing magnetocardiograms, which resemble electrocardiograms in many aspects, although the actual shape may vary depending on the position of the magnetic field sensor.

The term "myocardial disorder" relates to the condition of a non-healthy, i.e. a malfunctioning and/or damaged heart muscle. In particular, the term relates to a malfunctioning and/or damaged heart muscle, e.g. as a result of IHD.

The term "magnetic field sensor" as used herein means a sensor being able to measure magnetic fields, e.g. a gradiometer. The term comprises a magnetic field sensor (1-axis magnetic field sensor) being designed to only measure one component of the magnetic field, e.g. the z-component, or a magnetic field sensor (2- or 3-axis magnetic field sensor) being able to measure two or all three orthogonal components (x-, y- and z-component) of the magnetic field. SQUIDs ("superconducting quantum interference devices"), e.g. having axial gradiometers as pickup coil, are preferred as sensors.

As used herein, unless not stated otherwise or circumstances do not indicate otherwise, a reference to an x-axis in relation to the heart or the body of a human being corresponds to a reference to a right-to-left axis, a reference to an y-axis corresponds to a reference to a head-to-foot axis, and a reference to the z-axis corresponds to a reference to a anteroposterior axis. The term "z-component" of a magnetic field thus, for example, refers to the magnetic field component in the direction of the anteroposterior axis. The coordinates comprising a x-, y- and z-axis as explained above may also be referred to as "standard heart coordinates".

The term "sinus rhythm" relates to the rhythmic sequence of contractions of the heart during the cardiac cycle (heart beat) involving depolarization and repolarization of the atria and ventricles. In the sinus rhythm, depolarisation of the cardiac muscle begins at the sinoatrial node (also called sinus node or SA node) situated in the upper part of the wall of the right atrium, is conducted through the atrioventricular node (AV node) between the atria and ventricles to the bundle of His, the bundle branches and the Purkinje fibres. Repolarisation of the ventricles ends the cycle. Depolarisation and repolarisation of the atria and ventricles are reflected by three typical waves or wave complexes on a typical electrocardiogram (ECG), P-wave, QRS complex and T-wave. In the ECG, the P-wave is usually considered to represent atrial depolarization, the QRS complex the depolarization of the right and left ventricles, and the T-wave the repolarization of the ventricles.

The term "subject" as used herein refers preferably to a vertebrate, further preferred to a mammal, and most preferred to a human.

The term "measuring a component of the magnetic field at a plurality of positions above the heart" means measuring a component, e.g. the z-component, of the magnetic field at several positions which are, preferably evenly, spaced apart from each other, e.g. by using an array of magnetic field sensors, for example 32, 64 or 128 magnetic field sensors. The term "above the heart" is not to be construed as being constricted to a position in relation to the earth's gravitation field.

It is to be noted that a possible inherent sensor offset is, of course, taken into account when determining the monopolarity, i.e. a baseline will be set before computing the monopolarity.

The measurement of the magnetic field component may be performed under resting conditions, e.g. under conditions where the subject rests on a bed or suchlike, or under stress conditions, i.e. under conditions of physical stress, e.g. during exercises performed with an ergometer or suchlike.

In the method of the invention the monopolarity of the magnetic field is calculated by the following formula (1)

$$M = \frac{\left|\sum b_i\right|}{\sum |b_i|} \qquad (1),$$

wherein M is the monopolarity, $i$ the sensor index and $b_i$ the magnetic field gradient measured by the $i$-th magnetic field sensor. As an example, in case of e.g. 64 magnetic field sensors ($i$ = 1-64) measuring e.g. the z component of the magnetic field, the data measured by each of the 64 sensors is summed up, and the absolute value of this sum is then divided by the sum of the absolute values of each of the sensors.

The above formula provides a monopolarity score lying between 0 (zero, no monopolarity) and 1 (one, complete monopolarity). A perfect dipolar magnetic field would thus have a zero score, whereas any magnetic field deviating from a dipolar shape and having a more monopolar shape would have a score greater than zero. In reality, however, most magnetic fields generated by heart tissue deviate from a perfect dipolar shape and a zero score would be observed only in rare cases. Further, a positive score, i.e. a score greater zero, does not necessarily point to a malfunction of the heart tissue. In general, however, a comparatively high score is indicative of a possible malfunctioning of the heart muscle. Also in general, a higher score points to a more severe condition or form of a disorder. The inventors have, for example, found that a score of 0.8 or higher, in particular under stress conditions, e.g. during an ergometer stress-test, may be indicative of cardiac ischemia. An elevated score during rest may e.g. also point to other disorders, such as acute coronary syndrome or inflamed cardiac tissue. The skilled person may easily perform experiments in order to relate an elevated score, be it at rest and/or under stress conditions, with a myocardial disorder.

In a further preferred embodiment of the magnetocardiographic method of the invention, the monopolarity of the magnetic field is calculated over more than one predefined time period, for example over two, three, four or more time periods (intervals) during the ST-T segment of the sinus rhythm of the heart. The time periods may be consecutive intervals having the same or different lengths, preferably identical lengths, or time periods being separated by intervals. The intervals may cover the whole ST-T segment or only a portion thereof. The ST-T segment, or a portion thereof, may, for example, be split into four consecutive intervals of equal length, and an individual score be calculated for each interval. According to a preferred embodiment the ST-T segment, or a portion thereof, is thus separated into e.g. four consecutive subsegments, preferably of equal length, i.e. duration, and to calculate the monopolarity for each subsegment individually. In another preferred embodiment the ST-T segment is split into two subsegments, the first covering the portion of the ST-T segment from the end of the S wave to the maximum of the T wave (Tmax), or at least a portion thereof, and the second covering the ST-T segment from Tmax to Tend.

Although it is possible to take any component, i.e. the x-, y- or z-component, of the magnetic field for the determination of its monopolarity, it is preferred to use the z-component for this purpose. The z-component is the component usually measured by magnetocardiography. Using the z-component does not mean that only one-axis magnet field sensors, e.g. gradiometers measuring only z-component, can be implemented. Rather, also 3-axis magnet field sensors measuring the three orthogonal components of the magnetic field can be used, and only the data measured for the z-component are used for the calculation of the monopolarity of the magnetic field.

In the method of the invention the determination of the monopolarity of the magnetic field is computer implemented. The method is completely automated, and the score is computed from the data measured by means of a computer program running e.g. on a personal computer or another programmable apparatus. All steps necessary for determining the monopolarity of the magnetic field at a point in time of the ST-T interval or over at least one, preferably two or more time intervals being part of the ST-T interval are processed automatically. This includes, for example, the identification of the ST-T interval.

In an especially preferred embodiment of the invention the magnetocardiographic method thus comprises the following steps:

    a) Measuring a component of the heart magnetic field at a plurality of positions above the heart of a subject using a plurality of magnetic field sensors during at least part of the cardiac cycle comprising the ST-T segment of the sinus rhythm of the heart, and

b1) Identifying the ST-T segment of the sinus rhythm of the heart using a computer running a computer program being configured to identify the ST-T segment based on the data measured by the plurality of magnetic field sensors, and

b2) Determining the monopolarity of the magnetic field at at least one given point in time or over at least one given time period during the ST-T segment of the sinus rhythm of the heart using a computer running a computer program being configured to determine the monopolarity of the magnetic field, and using the magnetic field data measured by the plurality of magnetic field sensors, the monopolarity of the magnetic field representing a measure for the deviation of the magnetic field from a dipolar shape.

In a second aspect the invention also relates to a magnetocardiographic system, comprising a plurality of magnetic field sensors being able to measure a component of the magnetic field generated by the heart, and a calculation unit being configured to determine, from the magnetic field data measured by the plurality of magnetic field sensors, the monopolarity of the magnetic field at at least one given point in time or over at least one given time period during at least part of the cardiac cycle comprising the ST-T segment of the sinus rhythm of the heart, the monopolarity of the magnetic field representing a measure for the deviation of the magnetic field from a dipolar shape.

The calculation unit may, for example, be an integrated circuit, a Personal Computer or other programmable apparatus, or part thereof, being able to perform a calculation. In a preferred embodiment of the second aspect of the invention, the magnetocardiographic system comprises a Personal Computer running a program being configured to determine the monopolarity of the magnetic field. The magnetocardiographic system of the invention may thus be composed of a magnetocardiograph being designed and configured to measure, with a plurality of magnetic field sensors, e.g. SQUID sensors coupled to axial gradiometers being configured to measure the z-component of a magnetic field, the magnetic field of the heart of a subject, and a Personal Computer connected to the magnetocardiograph and configured to acquire and process the magnetic field data and to determine, from the magnetic field data, the monopolarity of the magnetic field, e.g. by using a suitable computer program calculating e.g. a score representing a measure for the monopolarity of the magnetic field. The determination of the monopolarity of the magnetic field may also comprise the identification of the ST-T segment.

The calculation unit is configured to calculate the monopolarity of the magnetic field according to the following formula (1)

$$M = \frac{\left| \sum b_i \right|}{\sum \left| b_i \right|} \quad (1),$$

wherein M is the monopolarity, $i$ the sensor index and $b_i$ the magnetic field gradient measured by the $i$-th magnetic field sensor.

In a preferred embodiment of the magnetocardiographic system according to the invention the calculation unit is configured to calculate the monopolarity of the magnetic field over more than one given time period, further preferably over two, three, four or more time periods. Preferably, the calculation unit is configured to calculate the monopolarity of the magnetic field over more than one predefined time period, for example over two, three, four or more time periods during the ST-T segment of the sinus rhythm of the heart. The time periods may be consecutive intervals having the same or different lengths, preferably identical lengths, or time periods being separated by intervals. As an example, the calculation unit may be configured to split the ST-T segment into four consecutive intervals of equal length, and to calculate an individual score for each interval, or to split the ST-T segment into two consecutive intervals, the first comprising the ST segment and the portion of the T wave until Tmax, the second comprising the T wave from Tmax to Tend.

Preferably, the plurality of magnetic field sensors of the magnetocardiographic system of the invention is able to measure the z-component of the magnetic field. The magnetic field sensors may, for example, be axial first- or second-order gradiometers coupled to a SQUID and oriented in a manner to be able to measure the z-component of the magnetic field, i.e. the component perpendicular to the x-y plane (frontal plane) according to standard heart coordinates. Such sensors are known to the skilled person.

In the following the invention is further described for illustration purposes only by way of the attached figures.

Figure 1 Schematic drawing of a magnetocardiogram.

Figure 2 Schematic isocontour drawings of an exemplary dipolar (A) and monopolar (B) magnetic field. Solid lines represent positive magnetic field values, dashed lines negative values.

Figure 3 Magnetic isofield contour maps of the ST-T segment of a healthy (A) subject and (B) a subject having cardiac ischemia.

Figure 4 Magnetic isofield contour map of the ST-T segment of a healthy subject (C), corresponding average monopolarity scores for four subsegments of the ST-T segment during rest and under stress (D), monopolarity score during time (B), and magnetic field data of each magnetic field sensor during the ST-T segment (A).

Figure 5 Magnetic isofield contour map of the ST-T segment of a subject afflicted with ischemia (C), corresponding average monopolarity scores for four subsegments of the ST-T segment during rest and under stress (D), monopolarity score during time (B), magnetic field data of each magnetic field sensor during the ST-T segment (A).

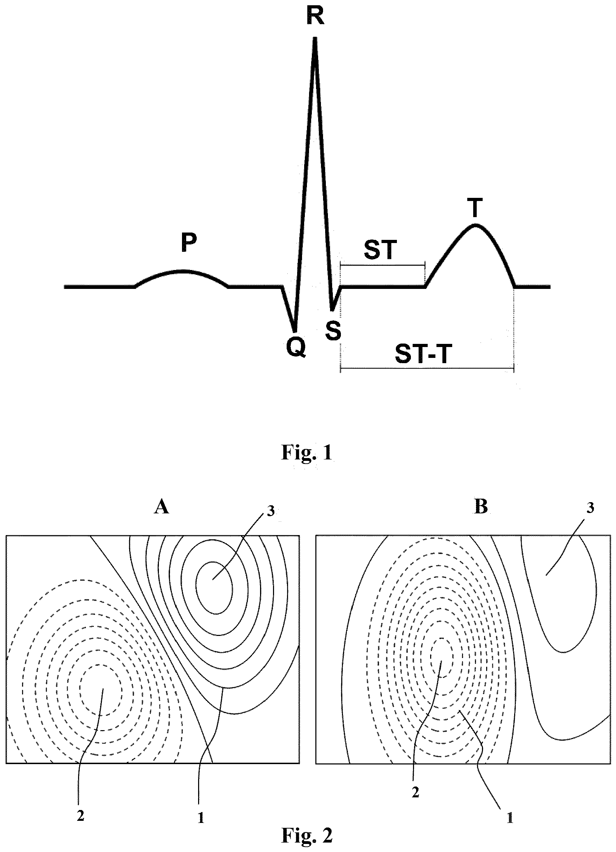

Figure 1 shows schematically an idealized magnetocardiogram of the cardiac cycle of a normal healthy subject. It is to be noted that only the data of one sensor at a specific position is depicted. Magnetocardiograms differ depending on the position of the sensor in relation to the magnetic field. Fig. 1 depicts P-wave, Q-R-S-complex and T-wave, which are well known to the skilled person. The ST segment and ST-T segment are also marked.

Figure 2 shows exemplary line drawings of a magnetic field having an essentially dipolar shape (A) and of a magnetic field having an essentially monopolar shape (B). The lines 1 are isolines (step size from one line to the next line is constant). Solid lines 1 represent positive values, dashed lines 1 negative values. The magnetic field depicted in Fig. 2A has an essentially dipolar shape with a distinct negative peak 2 and a distinct positive peak 3. In contrast, the magnetic field depicted in Fig. 2B has an essentially monopolar shape with only a distinct negative peak 2, but no distinct or with only a large diffused positive "peak" 3.

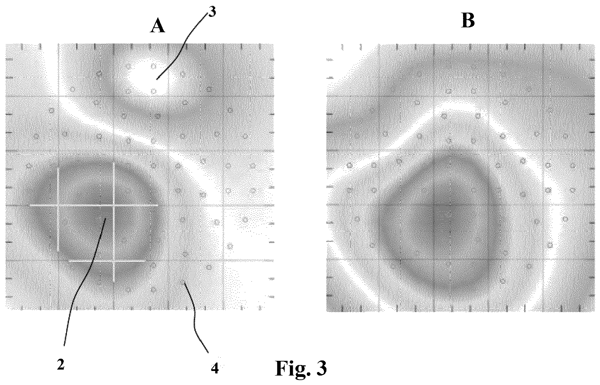

Figure 3 shows magnetic isofield contour maps (isofield maps, isocontour maps) of the ST-T segment of a healthy (A) subject and (B) of a subject having ischemia, measured with a magnetocardiographic system according to the invention. The isocontour maps were computed from the magnetic field data measured by a plurality of magnetic field sensors 4 for the z-component of the heart magnetic field. In this example, 64 magnetic field sensors 4, axial gradiometers coupled to a SQUID and arranged in an essentially circular array, were used. Figure 3A shows a normal isocontour map during the ST-T segment of a healthy person. The map shows the essentially dipolar shape of the magnetic field, with a positive peak 3 and a negative peak 2. Figure 3B shows an isocontour map during the ST-T segment of a person afflicted with an ischemic heart disease (IHD). It can readily be seen from the figure that there is essentially only one pole, namely a negative pole 2 in this case. Consequently, the magnetic field has an essentially monopolar shape.

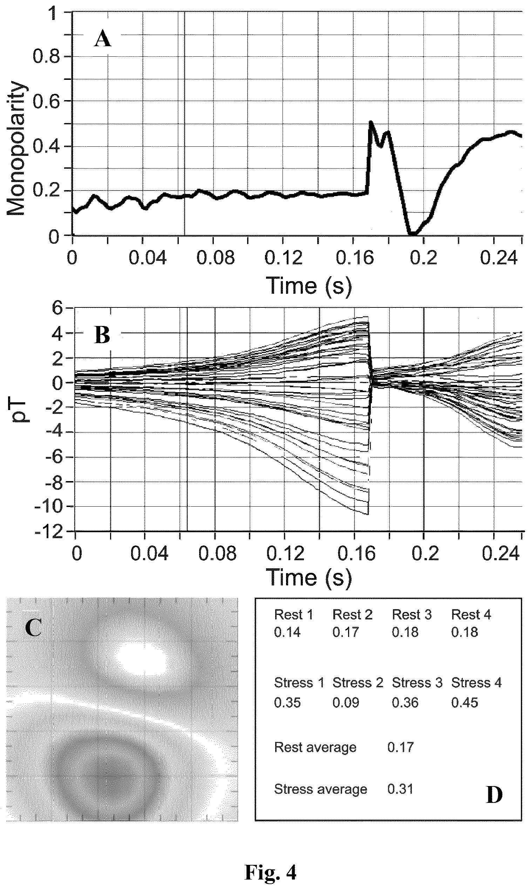

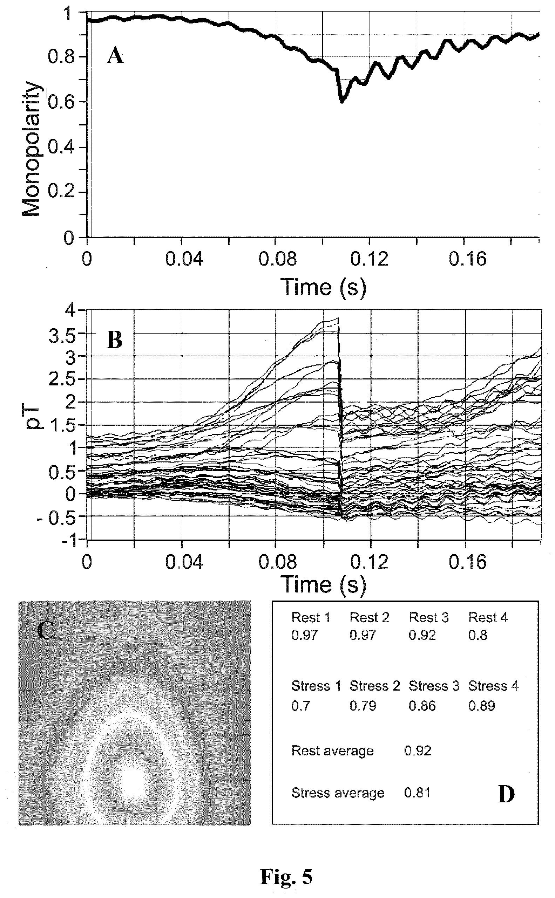

Figure 4 shows magnetic field data and monopolarity scores for a healthy person, whereas Figure 5 shows corresponding data for a subject suffering from IHD. In Figs. 4C and 5C isocontour maps similar to those in Fig. 3 are depicted. Again, the isocontour map of the healthy person (Fig. 4) shows an essentially dipolar shape, whereas the isocontour map of the person suffering from cardiac ischemia (Fig. 5) shows an essentially monopolar shape.

Figures 4 and 5 also show monopolarity scores (M) calculated according to the following formula (1):

$$M = \frac{\left|\sum b_i\right|}{\sum \left|b_i\right|} \tag{1}$$

Average monopolarity scores were independently calculated for four directly consecutive subsegments of the ST-T segment (Fig. 4D, 5D). The subsegments were of equal length (i.e. duration). Further, the monopolarity scores were calculated under rest (subject resting on a bed) and under stress (subject lying on a bed, but performing exercises with an ergometer placed at the end of the bed). Further, the monopolarity was also averaged over the total ST-T segment. Again, a "rest score" and a "stress score" were determined.

As can be seen from figure 4D the average monopolarity of the healthy person is 0.17 at rest, and 0.31 under stress. In contrast, the monopolarity scores of the subject having IHD (figure 5D) are much higher, both at rest and under stress conditions. At rest, the monopolarity score of the subject having IHD is 0.92 on average. Under stress, the monopolarity score is 0.81 on average. In figures 4B and 5B graphs showing the development of the monopolarity score over time are depicted. In figures 4A and 5A magnetic field data of each of the 64 sensors are depicted, showing that the magnetic field of the healthy person gives negative and positive signals corresponding to an essentially dipolar shape, whereas the magnetic field data of the person having IHD are mainly in the positive range. It is to be noted that, in figures 4A, B and 5A, B only values measured during the end phase of the ST segment and the T wave until Tmax are depicted, and that the data measured during this interval under stress conditions are directly appended to the values measured at rest. Therefore, the left part of the plots show the sensor data measured during the end of the ST segment and part of the T wave until Tmax at rest, whereas the right part of the plots show the sensor data measured during the end of the ST segment and part of the T wave until Tmax under stress.

References

[1] David Cohen, JC Norman, F Molokhia, and W Hood. Magnetocardiography of direct currents: St segment and baseline shifts during experimental myocardial infarction. Science, 172(3990):1329-1333, 1971.
[2] Helena Hänninen, Panu Takala, Markku Mäkijärvi, Juha Montonen, Petri Korhonen, Lasse Oikarinen, Jukka Nenonen, Toivo Katila, and Lauri Toivonen. Detection of exercise-induced myocardial ischemia by multichannel magnetocardiography in single vessel coronary artery disease. Annals of non-invasive electrocardiology, 5(2):147-157, 2000.

[3] Hyun Kyoon Lim, Namsik Chung, Kiwoong Kim, Young-Guk Ko, Hyukchan Kwon, Yong-Ho Lee, Jin-Bae Kim, Jung Rae Cho, Jin-Mok Kim, In-Seon Kim, et al. Reproducibility of quantitative estimate of magnetocardiographic ventricular depolarization and repolarization parameters in healthy subjects and patients with coronary artery disease. Annals of biomedical engineering, 35(1):59-68, 2007.

[4] Jaakko Malmivuo and Robert Plonsey. Bioelectromagnetism: principles and applications of bioelectric and biomagnetic fields. Oxford university press, 1995.

[5] Kirsten Tolstrup, Bo E Madsen, Jose A Ruiz, Stephen D Greenwood, Judeen Camacho, Robert J Siegel, H Caroline Gertzen, J-W Park, and Peter A Smars. Non-invasive resting magnetocardiographic imaging for the rapid detection of ischemia in subjects presenting with chest pain. Cardiology, 106(4):270-276, 2006.

[6] Satsuki YAMADA and Iwao YAMAGUCHI. Magnetocardiograms in clinical medicine: unique information on cardiac ischemia, arrhythmias, and fetal diagnosis, internal Medicine, 44(1):1-19, 2005.

[7] Yingmei Li, Zaiqian Che, Weiwei Quan, Rong Yuan, Yue Shen, Zongjun Liu, Weiqing Wang, Huigen Jin, Guoping Lu, Diagnostic outcomes of magnetocardiography in patients with coronary artery disease, Int J Clin Exp Med 2015;8(2):2441-2446.

[8] E. A. Perez Alday, C. Zhang, M. A. Colman, H. Ni, Z. Gan and H. Zhang, "Comparison of electric- and magnetic- cardiograms produced by myocardial ischemia in models of the human ventricle and torso", Computing in Cardiology Conference (CinC), Nice, 2015, pp. 517-520 (doi: 10.1109/CIC.2015.7410961).

[9] Brockmeier K, Schmitz L, Bobadilla Chavez JD, Burghoff M, Koch H, Zimmermann R, Trahms L., Magnetocardiography and 32-lead potential mapping: repolarization in normal subjects during pharmacologically induced stress, J Cardiovasc Electrophysiol. 1997 Jun;8(6):615-26 (doi: 10.1111/j.1540-8167.1997.tb01824.x).

[10] S. Ebmeyer, I. Chaikovsky, B. Hailer, R. Erbel, H. Wojczik, M. Budnyk, R. Simon, Predictive value of the magnetocardiogram for location of regional ischemia or infarction as detected by quantitative analysis of the coronary arteriogram, International Congress Series 1300 (2007), 463-467 (doi:10.1016/j.ics.2007.02.008).

**Claims**

1. Magnetocardiographic method, the method comprising the following steps:

   a) Measuring a component of the heart magnetic field at a plurality of positions above the heart of a subject using a plurality of magnetic field sensors during at least part of the cardiac cycle comprising the ST-T segment of the sinus rhythm of the heart, and
   b) Determining, using the magnetic field data measured by the plurality of magnetic field sensors, the monopolarity of the magnetic field at least one given point in time or over at least one given time period during the ST-T segment of the sinus rhythm of the heart,

   wherein the determination of the monopolarity of the magnetic field is computer implemented,
   **characterized in that** the monopolarity of the magnetic field represents a measure for the deviation of the magnetic field from a dipolar shape, and **in that** the monopolarity of the magnetic field is calculated by the following formula (1)

   $$M = \frac{\left|\sum b_i\right|}{\sum |b_i|} \qquad (1),$$

   wherein M is the monopolarity, $i$ the sensor index and $b_i$ the magnetic field gradient measured by the $i$-th magnetic field sensor.

2. Magnetocardiographic method according to claim 1, wherein the monopolarity of the magnetic field is calculated over more than one given time period, further preferably over two, three, four or more time periods during the ST-T segment of the sinus rhythm of the heart.

3. Magnetocardiographic method according to one of the preceding claims, wherein the component of the magnetic field is the z-component.

4. Magnetocardiographic method according to one of the preceding claims, wherein the determination of the monopolarity of the magnetic field further comprises the following step: b1) Identifying the ST-T segment of the sinus rhythm of the heart based on the data measured by the plurality of magnetic field sensors.

5. Magnetocardiographic system, comprising a plurality of magnetic field sensors being able to measure a component of the magnetic field generated by the heart, and a calculation unit being configured to determine, from the magnetic field data measured by the plurality of magnetic field sensors, the monopolarity of the magnetic field at at least one given point in time or over at least one given time period during at least part of the cardiac cycle comprising the ST-T segment of the sinus rhythm of the heart, **characterized in that** the monopolarity of the magnetic field represents a measure for the deviation of the magnetic field from a dipolar shape, and **in that** the calculation unit is configured to calculate the monopolarity of the magnetic field by the following formula (1)

$$M = \frac{\left|\sum b_i\right|}{\sum |b_i|} \qquad (1),$$

wherein M is the monopolarity, $i$ the sensor index and $b_i$ the magnetic field gradient measured by the $i$-th magnetic field sensor.

6. Magnetocardiographic system according to claim 5, wherein the calculation unit is configured to calculate the monopolarity of the magnetic field over more than one given time period, further preferably over two, three, four or more time periods.

7. Magnetocardiographic system according to one of claims 5 or 6, wherein the plurality of magnetic field sensors is able to measure the z-component of the magnetic field.

8. Magnetocardiographic system according to one of claims 5 to 7, comprising a computer being or comprising the calculation unit.

**Patentansprüche**

1. Magnetkardiographisches Verfahren, wobei das Verfahren die folgenden Schritte umfasst:

a) Messen einer Komponente des Herzmagnetfeldes an mehreren Positionen oberhalb des Herzens einer Person unter Verwendung mehrerer Magnetfeldsensoren während mindestens eines Teils des Herzzyklus, der den ST-T-Abschnitt des Sinusrhythmus des Herzens umfasst, und
b) Bestimmen der Monopolarität des Magnetfeldes zu mindestens einem bestimmten Zeitpunkt oder über mindestens eine bestimmte Zeitspanne während des ST-T-Abschnitts des Sinusrhythmus des Herzens unter Verwendung der von den mehreren Magnetfeldsensoren gemessenen Magnetfelddaten des Herzens, wobei die Bestimmung der Monopolarität des Magnetfeldes computergestützt erfolgt,

**dadurch gekennzeichnet, dass** die Monopolarität des Magnetfeldes ein Maß für die Abweichung des Magnetfeldes von einer dipolaren Form repräsentiert,
und dass die Monopolarität des Magnetfeldes durch die folgende Formel (1) berechnet wird

$$M = \frac{\left|\sum b_i\right|}{\sum |b_i|} \qquad (1),$$

wobei M die Monopolarität, $i$ der Sensorindex und $b_i$ der durch den $i$-ten Magnetfeldsensor gemessene Magnetfeldgradient ist.

2. Magnetkardiographisches Verfahren nach Anspruch 1, wobei die Monopolarität des Magnetfeldes über mehr als eine bestimmte Zeitspanne, weiter bevorzugt über zwei, drei, vier oder mehr Zeitspannen während des ST-T-Abschnitts des Sinusrhythmus des Herzens berechnet wird.

3. Magnetkardiographisches Verfahren nach einem der vorhergehenden Ansprüche, wobei die Komponente des Magnetfeldes die z-Komponente ist.

4. Magnetkardiographisches Verfahren nach einem der vorhergehenden Ansprüche, wobei die Bestimmung der Monopolarität des Magnetfeldes ferner den folgenden Schritt umfasst: b1) Identifizieren des ST-T-Abschnitts des Sinusrhythmus des Herzens basierend auf den von den mehreren Magnetfeldsensoren gemessenen Daten.

5. Magnetkardiographisches System, umfassend mehrere Magnetfeldsensoren, die in der Lage sind, eine Komponente des vom Herzen erzeugten Magnetfeldes zu messen, und eine Recheneinheit, die dahingehend konfiguriert ist, aus den von den mehreren Magnetfeldsensoren gemessenen Magnetfelddaten die Monopolarität des Magnetfeldes zu mindestens einem bestimmten Zeitpunkt oder über mindestens eine bestimmte Zeitspanne während mindestens eines Teils des Herzzyklus, der den ST-T-Abschnitt des Sinusrhythmus des Herzens umfasst, zu bestimmen, **dadurch gekennzeichnet, dass** die Monopolarität des Magnetfeldes ein Maß für die Abweichung des Magnetfeldes von einer dipolaren Form repräsentiert, und dass die Recheneinheit dahingehend konfiguriert ist, die Monopolarität des Magnetfeldes durch die folgende Formel (1) zu berechnen

$$M = \frac{\left|\sum b_i\right|}{\sum |b_i|} \qquad (1),$$

wobei M die Monopolarität, *i* der Sensorindex und $b_i$ der durch den *i*-ten Magnetfeldsensor gemessene Magnetfeldgradient ist.

6. Magnetkardiographisches System nach Anspruch 5, wobei die Recheneinheit dahingehend konfiguriert ist, die Monopolarität des Magnetfeldes über mehr als eine bestimmte Zeitspanne, weiter bevorzugt über zwei, drei, vier oder mehr Zeitspannen zu berechnen.

7. Magnetkardiographisches System nach einem der Ansprüche 5 oder 6, wobei die mehreren Magnetfeldsensoren in der Lage sind, die z-Komponente des Magnetfeldes zu messen.

8. Magnetkardiographisches System nach einem der Ansprüche 5 bis 7, umfassend einen Computer, der die Recheneinheit ist oder umfasst.

**Revendications**

1. Procédé magnétocartographique, le procédé comprenant les étapes suivantes :

a) la mesure d'une composante du champ magnétique du coeur à une pluralité de positions situées au-dessus du coeur d'un sujet en utilisant une pluralité de capteurs de champ magnétique pendant au moins une partie du cycle cardiaque comprenant le segment ST-T du rythme sinusoïdal coeur, et
b) la détermination, en utilisant les données de champ magnétique mesurées au moyen de la pluralité de capteurs de champ magnétique, la monopolarité du champ magnétique à au moins un point donné dans le temps ou pendant au moins une durée donnée pendant le segment ST-T du rythme sinusoïdal du coeur, dans lequel la détermination de la monopolarité du champ magnétique est mise en oeuvre par un ordinateur,

**caractérisé en ce que** la monopolarité du champ magnétique représente une mesure de la déviation du champ magnétique à partir d'une forme dipolaire, et **en ce que** la monopolarité du champ magnétique est calculée au moyen de la formule suivante (1)

$$M = \frac{\left|\sum b_i\right|}{\sum |b_i|} \qquad (1),$$

dans laquelle M représente la monopolarité, *i* l'indice de détection et $b_i$ le gradient de champ magnétique mesuré par le *l'i-ème* capteur de champ magnétique.

2. Procédé magnétocartographique selon la revendication 1, dans lequel la monopolarité du champ magnétique est calculée pendant plus d'une période donnée, de manière plus préférée pendant deux, trois, quatre ou plus durées pendant le segment ST-T du rythme sinusoïdal du coeur.

3. Procédé magnétocartographique selon l'une quelconque des revendications précédentes, dans lequel la composante du champ magnétique est la composante z.

4. Procédé magnétocartographique selon l'une quelconque des revendications précédentes, dans lequel la détermination de la monopolarité du champ magnétique comprend en outre l'étape suivante :
b1) l'identification du segment ST-T du rythme sinusoïdal du coeur en fonction des données mesurées au moyen de la pluralité de capteurs de champ magnétique.

5. Système magnétocartographique, comprenant une pluralité de capteurs de champ magnétique étant capables de mesurer une composante du champ magnétique généré par le coeur, et une unité de calcul étant conçue pour déterminer, à partir des données de champ magnétique mesurées au moyen de la pluralité de capteurs de champ magnétique, la monopolarité du champ magnétique à au moins un point donné dans le temps ou pendant au moins une durée donnée pendant au moins une partie du cycle cardiaque comprenant le segment ST-T du rythme sinusoïdal du coeur, **caractérisé en ce que** la monopolarité du champ magnétique représente une mesure de la déviation du champ magnétique à partir d'une forme dipolaire, et **en ce que** l'unité de calcul est conçue pour calculer la monopolarité du champ magnétique au moyen de la formule suivante (1)

$$M = \frac{\left|\sum b_i\right|}{\sum |b_i|} \qquad (1),$$

dans laquelle M représente la monopolarité, *i* l'indice de détection et $b_i$ le gradient de champ magnétique mesuré par le l'i-ème capteur de champ magnétique.

6. Système magnétocartographique selon la revendication 5, dans lequel l'unité de calcul est conçue pour calculer la monopolarité du champ magnétique pendant plus d'une période donnée, de manière plus préférée pendant deux, trois, quatre ou plus durées.

7. Système magnétocartographique selon l'une quelconque des revendications 5 ou 6, dans lequel la pluralité de capteurs de champ magnétique est capable de mesurer la composante z du champ magnétique.

8. Système magnétocartographique selon l'une quelconque des revendications 5 à 7, comprenant un être informatique ou comprenant l'unité de calcul.

**Fig. 1**

**Fig. 2**

Fig. 3

**Fig. 4**

| Rest 1 | Rest 2 | Rest 3 | Rest 4 |
|--------|--------|--------|--------|
| 0.97 | 0.97 | 0.92 | 0.8 |

| Stress 1 | Stress 2 | Stress 3 | Stress 4 |
|----------|----------|----------|----------|
| 0.7 | 0.79 | 0.86 | 0.89 |

Rest average    0.92

Stress average    0.81    **D**

**Fig. 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 02017769 A2
- DE 10042138 A1

### Non-patent literature cited in the description

- **DAVID COHEN ; JC NORMAN ; F MOLOKHIA ; W HOOD.** Magnetocardiography of direct currents: St segment and baseline shifts during experimental myocardial infarction. *Science,* 1971, vol. 172 (3990), 1329-1333
- **HELENA HÄNNINEN ; PANU TAKALA ; MARKKU MÄKIJÄRVI ; JUHA MONTONEN ; PETRI KORHONEN ; LASSE OIKARINEN ; JUKKA NENONEN ; TOIVO KATILA ; LAURI TOIVONEN.** Detection of exercise-induced myocardial ischemia by multichannel magnetocardiography in single vessel coronary artery disease. *Annals of noninvasive electrocardiology,* 2000, vol. 5 (2), 147-157
- **HYUN KYOON LIM ; NAMSIK CHUNG ; KIWOONG KIM ; YOUNG-GUK KO ; HYUKCHAN KWON ; YONG-HO LEE ; JIN-BAE KIM ; JUNG RAE CHO ; JIN-MOK KIM ; IN-SEON KIM et al.** Reproducibility of quantitative estimate of magnetocardiographic ventricular depolarization and repolarization parameters in healthy subjects and patients with coronary artery disease. *Annals of biomedical engineering,* 2007, vol. 35 (1), 59-68
- **JAAKKO MALMIVUO ; ROBERT PLONSEY.** Bioelectromagnetism: principles and applications of bioelectric and biomagnetic fields. Oxford university press, 1995
- **KIRSTEN TOLSTRUP ; BO E MADSEN ; JOSE A RUIZ ; STEPHEN D GREENWOOD ; JUDEEN CAMACHO ; ROBERT J SIEGEL ; H CAROLINE GERTZEN ; J-W PARK ; PETER A SMARS.** Non-invasive resting magnetocardiographic imaging for the rapid detection of ischemia in subjects presenting with chest pain. *Cardiology,* 2006, vol. 106 (4), 270-276
- **SATSUKI YAMADA ; IWAO YAMAGUCHI.** Magnetocardiograms in clinical medicine: unique information on cardiac ischemia, arrhythmias, and fetal diagnosis, internal. *Medicine,* 2005, vol. 44 (1), 1-19
- **YINGMEI LI ; ZAIQIAN CHE ; WEIWEI QUAN ; RONG YUAN ; YUE SHEN ; ZONGJUN LIU ; WEIQING WANG ; HUIGEN JIN ; GUOPING LU.** Diagnostic outcomes of magnetocardiography in patients with coronary artery disease. *Int J Clin Exp Med,* 2015, vol. 8 (2), 2441-2446
- **E. A. PEREZ ALDAY ; C. ZHANG ; M. A. COLMAN ; H. NI ; Z. GAN ; H. ZHANG.** Comparison of electric- and magnetic- cardiograms produced by myocardial ischemia in models of the human ventricle and torso. *Computing in Cardiology Conference (CinC),* 2015, 517-520
- **BROCKMEIER K ; SCHMITZ L ; BOBADILLA CHAVEZ JD ; BURGHOFF M ; KOCH H ; ZIMMERMANN R ; TRAHMS L.** Magnetocardiography and 32-lead potential mapping: repolarization in normal subjects during pharmacologically induced stress. *J Cardiovasc Electrophysiol.,* June 1997, vol. 8 (6), 615-26
- **S. EBMEYER ; I. CHAIKOVSKY ; B. HAILER ; R. ERBEL ; H. WOJCZIK ; M. BUDNYK ; R. SIMON.** Predictive value of the magnetocardiogram for location of regional ischemia or infarction as detected by quantitative analysis of the coronary arteriogram. *International Congress Series,* 2007, vol. 1300, 463-467